# EUROPEAN PATENT SPECIFICATION

(11) **EP 2 157 184 B1**
(45) Date of publication and mention of the grant of the patent: **18.11.2020**
(21) Application number: 07747199.3
(22) Date of filing: 19.04.2007
(51) Int. Cl.: C12R 1/465, C12R 1/01, C12P 7/24, C12N 11/08

(54) **PROCESS FOR PRODUCING VANILIN FROM MICROORGANISMS IMMOBILIZED BY SURFACE CULTURE**
VERFAHREN ZUR HERSTELLUNG VON VANILLIN MITTELS OBERFLÄCHENKULTUR DURCH IMMOBILISIERTE MIKROORGANISMEN
PROCÉDÉ DE PRODUCTION DE VANILLINE À PARTIR DE MICRO-ORGANISMES IMMOBILISÉS PAR CULTURE DE SURFACE

(43) Date of publication of application: 24.02.2010
(73) Proprietor: Laboratorios Minkab, S.A. de C.V., Tlalnepantla, C.P. 54090 (MX)
(72) Inventor: ASAFF TORRES, Ali, C.P. 83170 Hermosillo, Sonora (MX); DE LA TORRE MARTÍNEZ, Mayra, Hermosillo, Sonora (MX); MACIAS OCHOA, Roberto Miguel, Delegación Benito Juárez, México D.F. (MX)
(74) Representative: Petraz, Gilberto Luigi
(86) International application number: PCT/MX2007/000053
(87) International publication number: WO 2008/130210

(56) References cited:
- EP-A1- 0 885 968
- EP-A1- 1 734 128
- WO-A1-2004/085663
- FR-A1- 2 647 119
- FR-A1- 2 733 763
- US-A- 5 866 380
- TORRE P. ET AL.: 'Bioconversion of ferulate into vanillin by Escherichia coli strain JM109/pBB1 in immobilized-cell reactor' ANNALS OF MICROBIOLOGY vol. 54, no. 4, 2004, pages 517 - 527, XP008122084
- Moustafa Y El-Naggar ET AL: "Effect of support materials on antibiotic MSW2000 production by immobilized Streptomyces violatus", Journal of general applied microbiology, vol. 49, 1 January 2003 (2003-01-01), pages 235-243, XP055246811,
- Sri Devi ET AL: "Production of cephamycin C in repeated batch operations from immobilized Streptomyces clavuligerus", Process Biochemistry, vol. 36, 1 January 2000 (2000-01-01), pages 225-231, XP055246803,
- Sergio De Jesus Romero Gomez: "Producción de lnvertasa por Aspergillus niger en Fermentación Líquida y Fermentacion Sólida", , 1 April 2010 (2010-04-01), pages 1-134, XP055472693, Retrieved from the Internet: URL:http://tesiuami.izt.uam.mx/uam/aspuam/ presentatesis.php?recno=2828&docs=UAM2828. PDF

## Description

### TECHNICAL FIELD OF THE INVENTION

The invention refers to a process to produce vanillin. Particularly, it refers to a process to produce vanillin by means of biotransformation of ferulic acid through immobilized *Streptomyces sertonii* ATCC 39116 in polyurethane foam starting from a surface culture, which is a simple embodiment of the solid-substrate culture.

### BACKGROUND OF THE INVENTION

Vanillin is a compound broadly used as a flavouring agent in food industry, as an aroma in the cosmetics industry, and as a precursor for the chemical synthesis of drugs in the pharmaceutical industry. Mostly, the vanillin is obtained by chemical synthesis starting from guaiacol and lignin, as described by Clark, "Vanillin" in Perf Flavor (1990) 15: 45-54. However, although the vanillin obtained by this method is suitable for use in the cosmetics and pharmaceutical industries, it can confront legislative problems in food industry. Also at the present time, the tendency among the consumers for products of natural origin is increasing, leaving aside the synthetic products. Until now, the marketed natural vanillin has been obtained only by extraction of the vanilla pods, although at a very high cost of the raw material.

During the last 13 years many efforts have been made for obtaining a natural vanillin by alternative biological processes, which use microorganisms (bacteria, yeasts, fungi), plant cells or their enzymatic systems.

In general, these biological processes involve the biotransformation of a suitable precursor into vanillin. Eugenol, isoeugenol, curcumin, some resins and ferulic acid have been pointed out as precursors. In most of the cases, the transformation yields are very low as it is detailed at the published revision: "H. Priefert et al., Appl. Microbiol. Biotechnol. (2001) 56: 296-314". Just in some cases, the fermentation processes that have been described in the last years offer high volumetric yields with an attractive economical potential.

For example, Blandine Audras and Joeelle More, in the international patent application WO-9634971, describe a process in submerged culture for the biotransformation of ferulic acid into vanillin by *Streptomyces setonii* (strain CNCM n°I-1555) with the microorganism immobilized in calcium alginate spherules. At this point concentrations between 0.65 and 0.90 g L-1 of vanillin were reached in a period of 3 days with a molar yield from 53% to 69%, with regards to the ferulic acid consumed. Additionally, 0.65 g L-1 of vanillic acid as fermentation subproduct were obtained. An economic advantage of this process is that the biocatalyst can be recycled.

Juergen Rabenhorst and Rudolf Hopp, in the European patent application EP-0405197, describe the isolation of a new strain of *Amycolatopsis* sp., which in submerged culture is able to transform 19.92 g L⁻¹ of ferulic acid into 11.5 g L⁻¹ of vanillin in 32 hours with a molar yield of 77.8%.

Andreas Muheim *et al.* in the United States Patent US-6,235,507 B1, disclose a process in submerged culture for the biotransformation of ferulic acid in vanillin by *Streptomyces setonii* strain ATCC 39116, reaching a vanillin concentration of 9 g L⁻¹ in a period of 26 hours of biotransformation, with a molar yield of 51%.

The enzymes and the corresponding genes involved in the biotransformation to vanillin have been recently characterized and used for their production (WO-9735999, US-20010014467A1). The growing knowledge of the enzymes involved in the metabolic routes of biotransformation offers new opportunities for the metabolic engineering and for the construction of genetically modified organisms (US-20030070188A1, US-20030092143A1, US-6,372,461B1, WO - 2004/006657A1).

However, the use of genetically modified organisms in the production of vanillin can be problematic due to the objections of the consumers to this type of products, especially in Europe.

In different scientific publications vanillin it is pointed out as an intermediary compound in the metabolic routes of the degradation of its precursors. Two publications refer concretely to the implication of vanillin in the degradation of ferulic acid. Toms and Wood, Biochemistry (1970) 9: 337-43, grew *Pseudomonas sp.* In ferulic acid and they elucidated the degradation route. The final product obtained was vanillic acid, whereby they deduced that vanillin is only an unstable intermediary of this route. Sutherland et al., Can. J. Microbiol. (1983) 29: 1253-57, studied the degradation of ferulic acid in *Streptomyces setonii* cultures, finding out that this microorganism is able to accumulate vanillin as a degradation product that can be subsequently transformed into vanillic acid, vanillyl alcohol and/or guaiacol. In both cases, the process of degradation of ferulic acid involves a unique step of deacetylation of this compound, which does not use CoA.

The ferulic acid used as substrate for the biotransformation is a very abundant compound in the nature, as it is a component of the cellular wall of many plant species such as rice, maize, sugar beet, among others. However, it does not exist in a free form, but forming glycosidic bonds with carbohydrate chains of the cellular wall, therefore hydrolytic methods, either enzymatic or alkaline methods, are used for releasing it. Birgit Michelsen *et al.,* United States Patent US-6,143,543 A1, describe an enzymatic method to obtain free ferulic acid. The International Patent Application WO-2004/110975 A1 discloses a process for the recovery and purification of the ferulic acid in its free form starting from the cooking water of maize, known as "nexayote" which results from the *nixtamal* (maize specially prepared for making *tortillas*) industry.

The biotransformation processes to obtain vanillin, up to here described, are carried out in submerged culture. Although this fermentation system allows taking a good control of the process, it involves high investment and operation costs due to its intrinsic characteristics and volumetric productivity.

During the last years there has been a great interest for processes in solid-substrate cultures, or their more simple embodiment, which is the surface culture, thanks to a better development of many microorganisms in these culture systems, a high specific growth rate, as well as high yields in the production of metabolites of commercial importance and enzymes (Papagianni et al., Food Technol. Biotechnol. (2001) 39: 319-326).

Therefore it is necessary to provide a process to produce vanillin in concentrations that are industrially interesting, by means of the biotransformation of ferulic acid with immobilized microorganisms using a surface culture.

### SUMMARY OF THE INVENTION

In view of previously described matter and with the purpose of giving solution to the encountered obstacles, an object of the invention is to offer a process biotransforming ferulic acid into vanillin, the process comprises the steps of: (a) immobilizing a microorganism of the strain *Streptomyces sertonii* ATCC 39116 in polyurethane foam; (b) adding a solution of ferulic acid or any salt thereof; (c) incubating to carry out the biotransformation reaction; (d) recovering the biotransformation solution obtained in step (c); and (e) extracting the vanillin from the biotransformation solution obtained in step (d).

### BRIEF DESCRIPTION OF THE FIGURES

The characteristic details of the invention are described in the following paragraphs together with the accompanying figures, which have the purpose of defining to the invention but without limiting its scope.
Figure 1 shows the *Streptomyces setonii* actinomycete grown and fixed in the polyurethane foam after its surface culturing.
Figure 2 shows the growth profiles of *Streptomyces setonii* in a submerged culture according to the state of the art (*µ* = 0.22 h⁻¹) and in a surface culture according to the invention (*µ* = 0.70 h⁻¹).
Figure 3 shows the analysis using High Performance Liquid Chromatography (HPLC) of the biotransformation medium at three moments from the beginning of the process. The obtained products are observed: 1) ferulic acid; 2) vanillin; 3) vanillyl alcohol; 4) vanillic acid; and 5) guaiacol.
Figure 4 shows the maximum concentration of vanillin reached during several biotransformation cycles and the volumetric productivity in each one.

### DETAILED DESCRIPTION OF THE INVENTION

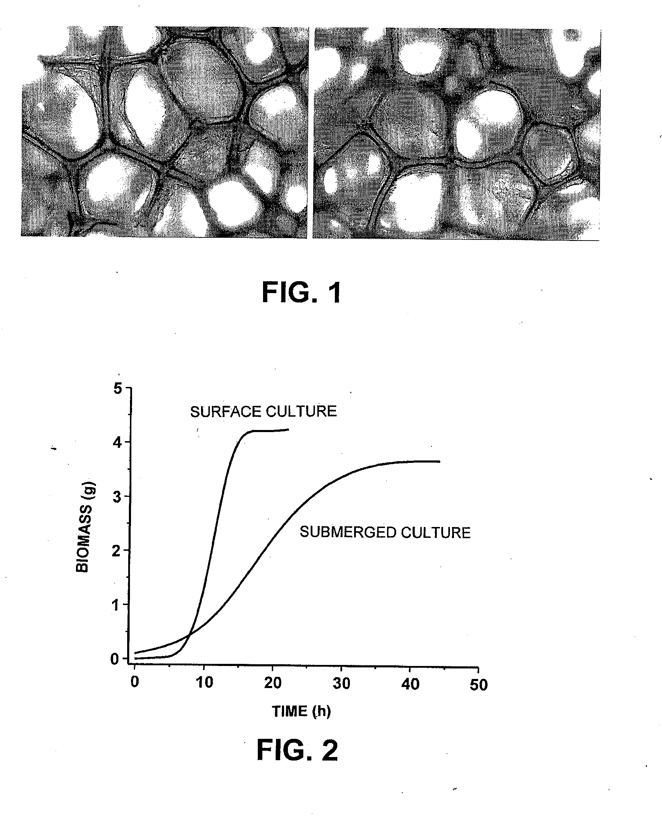

The invention contemplates a microbiological process with a high yield of biotransformation of ferulic acid into vanillin, that comprises initially the surface culture of microorganisms of the bacteria *Streptomyces setonii* ATCC 39116, in a nutrititious medium. The surface culture, which is a simple embodiment of the solid-substrate culture, comprises a system with the following characteristics: The aqueous culture medium, after being inoculated with the microorganism, is added to a polyurethane foam, which is a porous inert, compressible material, in which it is absorbed. Among the interstices of the foam, the inoculated nutritious medium forms very thin films having a thickness of from approximately 0.50µm to 0.90µm. In this way, a very compact system with a high contact surface is obtained, in which approximately 1g of foam, containing between 5mL and 50mL of the medium, preferably between 20mL and 35mL of the medium, offers a culture surface of from 3000cm² to 4000cm², just as described in the doctoral thesis of Romero-Gomez, Metropolitan Autonomous University (Iztapalapa campus), Mexico D.F. (2001) and in the Mexican Patent MX-178723, for the production of enzymes and other fungal metabolites. The foam has a density from 0.005 g mL⁻¹ to 0.070 g mL⁻¹; preferably from 0.015 g mL⁻¹ to 0.025 g mL⁻¹, cut into small cubes having a side length from 0.2 cm to 3.0 cm, preferably a side length from 0.4 cm to 0.9 cm, deposited in closed containers such as flasks or trays, with a bed height between 1 cm and 10 cm, preferably between 3 cm and 6 cm. The term "bed height", refers to the distance between the base and the maximum vertical level reached by the polyurethane cubes deposited in the container which contains them. The system is incubated at a temperature of from 30°C to 45°C, for a period from 6 hours to 35 hours, a period in which the microorganism is growing as a mycelium on the surface of the formed films, remaining fixed between the structures of compressible inert porous material, as it can be observed from figure 1. At the end of the fermentation, the biomass reaches a maximum and the carbon source, usually glucose, is run out. The residual nutrients such as the nitrogen source and salts of the culture medium in solution are then separated from the biomass that is fixed in the support by compressing the support. The remaining aqueous culture medium is discarded.

For the biotransformation process, a solution of ferulic acid or salts thereof with a concentration of approximately 5 g L⁻¹ to approximately 30 g L⁻¹, preferably of approximately 10 g L⁻¹ to approximately 20 g L⁻¹, with a pH between 7 and 9, preferably between 7.5 to 8.5, in a volume of 5 mL to 50mL, preferably from 20mL to 30mL per gram of inert support, is added to the polyurethane foam containing the immobilized microorganisms and which is free from the residual culture medium. The biotransformation is carried out to a temperature from 30°C to 45°C for a period of 7 to 48 hours. At the end of this stage, the whole precursor is substantially consumed, and the resulting vanillin is accumulated in the aqueous layers of the surface culture, in concentrations of approximately 3 g L⁻¹ to approximately 12 g L⁻¹, with a molar yield from 70% to 80%. In addition, vanillic acid, vanillyl alcohol and traces of guaiacol are produced, although in much smaller amounts. All the biotransformation products are recovered in solution by compression. The immobilized microorganisms in the support that is free from the aqueous medium, can be used again for carrying out a new biotransformation process, adding a fresh solution of ferulic acid. This process can be repeated cyclically between 2 times and 15 times, preferably between 6 and 10 times, with similar biotransformation yields.

It is important to finish the biotransformation process of the invention when the precursor is being depleted because afterwards, degradation of the formed vanillin into vanillic acid and vanillyl alcohol occurs. In other words, there are practically two clearly-defined stages, which consist on vanillin formation and vanillin degradation. It is very important to consider this fact if it is wanted to achieve an easier purification process and to assure a yield that is industrially interesting.

The recovery and purification of the vanillin is carried out by means of adsorption in activated coal or in synthetic resins of the family of the amberlite, starting from the solution obtained from the biotransformation process. The adsorbed vanillin is eluded with 95% ethanol, then the resulting solution is concentrated between 0.05 g mL⁻¹ and 0.5 g mL⁻¹, preferably between 0.1 g mL⁻¹ and 0.4 g mL⁻¹. Water is added to the concentrated solution, leading to the crystallization of vanillin. The above-described purification process is particularly important because it involves the use of a solvent that is entirely of natural origin.

As it has been pointed out previously, the appropriate combination of a novel fermentation system such as the surface culture, with exact fermentation conditions and an effective purification process, allow the biotransformation of ferulic acid or salts thereof into vanillin, with a high yield. The fermentation system is based on the surface culture of the strain ATCC 39116 of *Streptomyces sertonii,* in an appropriate culture medium.

In order to carry out the surface culture, an aqueous medium containing usual nutrients is used, said medium is absorbed by the polyurethane foam, which forms thin films among its interstices. The microorganism grows as a mycelium on the formed films. An appropriate culture medium contains a carbon source, a nitrogen source, inorganic salts and growth factors.

As carbon source, glucose or maltose is preferably used in a concentration from approximately 8 gL⁻¹ to approximately 40 g L⁻¹, preferably from approximately 15 g L⁻¹ to approximately 30 g L⁻¹. As nitrogen source, growth factors and trace elements, yeast extract is usually employed, in a concentration of from approximately 1 g L⁻¹ to approximately 15 g L⁻¹, preferably in a concentration of from approximately 3 g L⁻¹ to approximately 10 g L⁻¹. Additionally, a magnesium source and a phosphates buffer solution (from pH 7 to pH 8) are used.

The prepared culture medium is sterilized and then inoculated with *Streptomyces sertonii* ATCC 39116.

The used inoculum is obtained from the submerged culture of the microorganism in the same culture medium. The inoculum, with an age of 15 to 30 hours, preferably from 18 h to 24 h, is added to a volume from 3% to 6% (v/v). Subsequently, the culture means with the inoculum is added to the sterile polyurethane foam, where it is absorbed, in a volume from 5 mL to 50 mL per gram of foam, preferably from 20mL to 35 mL per gram of foam, thereby initiating the growth by surface culture. The fermentation is carried out at a temperature from 30°C to 45°C, with a duration of the growth period from 6 to 35 hours, preferably from 12 to 24 hours.

Once the growth phase is finished, the polyurethane foam containing the biomass that remained immobilized among the interstices is compressed, which allows draining the aqueous medium containing the residual nutrients. In such way, the foam again recovers its absorbent capacity, being fed afterwards with a ferulic acid solution having a concentration of from approximately 5 g L⁻¹ to approximately 30 g L⁻¹, preferably of from approximately 10 g L⁻¹ to approximately 20 g L⁻¹, with a pH between 7 and 9, preferably between 7.5 and 8.5. An appropriate amount of feeding is from 5 mL to 50 mL per gram of foam, preferably from 20mL to 35mL per gram of foam.

The biotransformation phase begins at the moment of the feeding and has a duration from 7 h to 48 h, preferably from 15 h to 28 h, after which practically all the substrate has been consumed and transformed into vanillin and some minor subproducts.

Once the biotransformation phase is finished, the foam containing the immobilized biomass, is again separated from the aqueous medium containing the vanillin by compression. The recovered solution drags a small amount of cellular material that is separated by centrifugation or filtration, in order to proceed afterwards with the recovery and purification process, just as it was described previously. The foam that is free from the aqueous medium is again ready to begin a new biotransformation cycle, by feeding a fresh solution of ferulic acid. The system maintains its efficiency and rate of conversion along 3 to 15 cycles, preferably 6 to 10 cycles, after which a loss of cellular viability, as well as a gradual washing of the biomass, take place.

It is important to point out that only the stage of the microorganism growth is carried out under sterile conditions, because later, when the culture medium is eliminated, there is no possibility of development of non-desired microorganisms. The same applies during the process of feeding the ferulic acid solution and recovering the vanillin in solution, since both compounds are toxic for a great variety of microorganisms, and only those microorganisms that have a highly specialized detoxification mechanism, such as *Streptomyces setonii,* are able to survive.

### ILLUSTRATIVE EXAMPLES OF THE INVENTION

The invention will now be described with reference to the following examples, which are only for the purpose of representing the way of carrying out the implementation of the principles of the invention.

### Example 1

Flasks of 250 mL, containing 1 g of polyurethane foam cut into small cubes, were prepared. 20 mL of culture medium were added to the flasks, which were previously inoculated with 0.8 mL of a preculture of *Streptomyces setonii* ATCC 39116, grown in an agitated flask at 37°C, at 190 min⁻¹, for 20 h. The culture medium contained 10 g L⁻¹ glucose, 4 g L⁻¹ yeast extract, 4 g L⁻¹ Na₂HPO₄, 1 g L⁻¹ KH₂PO₄, 0.2 g L⁻¹ MgSO₄ · 7H₂O, 0.2 g L⁻¹ NaCl and 0.05 g L⁻¹ CaCl₂· H₂O, with a pH of 7.2. The surface culture in the flasks was treated at 37 °C for 18 h, after which the glucose was consumed. After this stage, the polyurethane foam cubes were compressed inside a syringe, liberating the residual culture medium, which was discarded later. In order to start the biotransformation, 20 mL of a solution of 10 g L⁻¹ of ferulic acid at pH 7.2 was added to the biomass that was immobilized in the polyurethane foam, and placed again inside the flasks.

The biotransformation was carried out at 37 °C for 20 h, at the end of which the foam was pressed, liberating the biotransformation solution that was recovered with a content of 4.3 g L⁻¹ vanillin, 0.7 g L⁻¹ ferulic acid, 0.12 g L⁻¹ vanillic acid, 0.09 g vanillyl alcohol and traces of guaiacol. The calculated molar yield of conversion into vanillin was 60%. The cubes with the immobilized microorganisms were returned to the flasks, adding 20 mL of a fresh solution of 10 g L⁻¹ ferulic acid, maintaining the flasks at 37 °C for 24 h. The recovered biotransformation solution contained 6.1 g L⁻¹ vanillin, 0.39 g L⁻¹ ferulic acid, 0.06 g L⁻¹ vanillic acid, 0.04 g L⁻¹ vanillyl alcohol and traces of guaiacol. The resulting biotransformation yield was 81%. Additionally, 3 biotransformation cycles were repeated under the same temperature conditions and during the same period, obtaining results that were similar to the previous one. In the 5 biotransformation cycles, a total volume of 90 mL of solution was collected per flask.

### Example 2

12 L trays with lid were prepared, containing 25 g of polyurethane foam cut into small cubes. 500 mL of the culture medium previously inoculated with 20 mL of a preculture of *Streptomyces setonii* ATCC 39116, grown in an agitated flask at 37 °C, at 190 min⁻¹, for 20 h, were added to the trays. The culture medium contained 15 g L⁻¹ glucose, 6 g L⁻¹ yeast extract, 4 g L⁻¹ Na₂HPO₄, 1 g L⁻¹ KH₂PO₄, 0.2 g L⁻¹ MgSO₄ ·7H₂O, 0.2 g L⁻¹ NaCl and 0.05 g L⁻¹ CaCl₂· H₂O, with pH 7.2. The surface culture in the trays was treated at 37 °C for 24 h, at the end of which the glucose was consumed. After this stage, the polyurethane foam cubes were compressed inside the trays, liberating the residual culture medium, which was discarded. Afterwards, 500 mL of a solution of 10 g L⁻¹ ferulic acid at pH 7.2 was added.

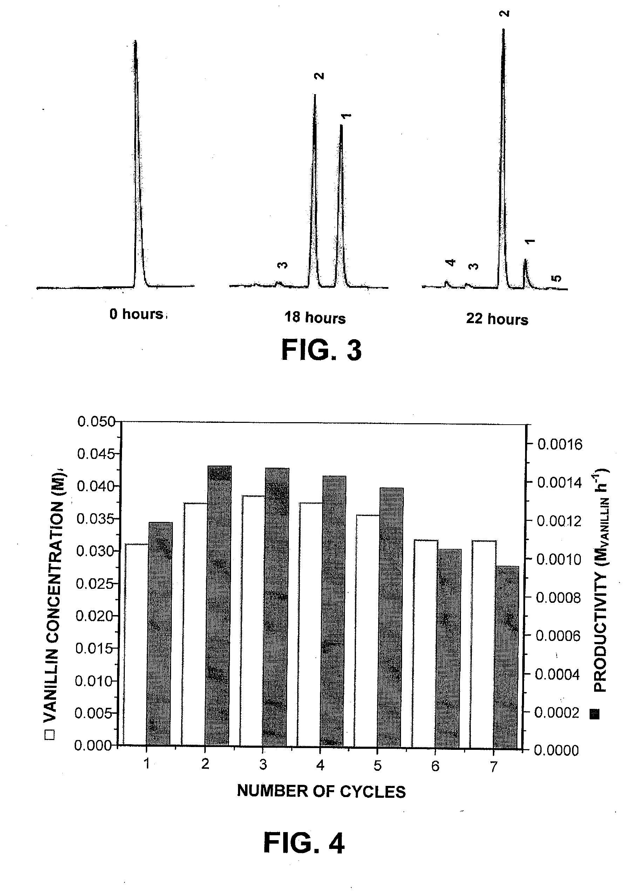

The biotransformation was carried out at 37° C for 24 h, at the end of which the biotransformation solution containing 4.71 g L⁻¹ vanillin, 0.63 g L⁻¹ ferulic acid, 0.07 g L⁻¹ vanillic acid, 0.06 g vanillyl alcohol and traces of guaiacol, was recovered similarly as in the previous example. The calculated molar yield of conversion into vanillin was 64 %. In a second cycle, 500 mL of a fresh solution of 10 g L⁻¹ ferulic acid was again added, mantaining the trays at 37 °C for 24 h. The recovered biotransformation solution had a content of 5.87 g L⁻¹ vanillin, 0.24 g L⁻¹ ferulic acid, 0.14 g L⁻¹ vanillic acid, 0.10 g L⁻¹ vanillyl alcohol and 0.04 g guaiacol. The conversion yield was 77 %. In addition, 5 biotransformation cycles were repeated under the same temperature conditions and for the same period, obtaining results that were similar to the previous one. In the 7 biotransformation cycles, a total volume of about 3.2 L of a solution containing 5.6 g L⁻¹ of vanillin was collected per tray. See Figure 4.

For the recovery and purification of vanillin, 100 g of activated coal were added to the biotransformation solution, leaving under agitation for 5 h, at the end of which the supernatant was discarded. The desortion was carried out with 100 mL of a 95% solution of ethanol under agitation for 3 h. The ethanolic solution was concentrated by evaporation, reducing its volume to 30 mL. Subsequently, 30 mL of water were added, leaving under rest for 12 hours, at the end of which the vanillin crystallized.

### Example 3

In another embodiment of the invention, 150 g of amberlite XAD-4 were used instead of the activated coal used in example 2, continuing with the same steps until reaching the crystallization of vanillin.

The advantages of the invention with respect to the state of the art can be summarized in the following items:
• The process allows the biotransformation of ferulic acid and accumulation of vanillin in concentrations that result industrially interesting (from about 3 g L⁻¹ to about 12 g L⁻¹). See figure 4.
• Thanks to the intrinsic advantages of the surface culture, the specific growth rate of *Streptomyces setonii* strain ATCC 39116 is increased up to three times, in comparison to its growth in submerged culture, therefore the time required for the growth of the microorganism is reduced. See Figure 2.
• The conversion process, compared to other processes carried out in submerged culture, is selective because the vanillin is almost the only product of biotransformation.
• The system employed allows fixing the microorganisms in an inert, porous, compressible support, whereby it is possible to reutilize them in a new biotransformation process (cycle) by fresh feeding of a ferulic acid solution.
• The operation costs related to the raw materials of the culture medium, as well as the auxiliary services, are reduced in a number of times equivalent to the amount of biotransformation cycles (from 3 cycles to 15 cycles) in comparison to a batch submerged culture.
• The process of the invention has a low technical complexity, therefore the costs of inversion in facilities and equipment are, for much, less than those required for a submerged culture process.
• The recovery and purification process is highly favored due to the fact that the solutions resulting from the biotransformation contain vanillin almost as the unique product, because the process is selective. Moreover, it does not contain inorganic salts or other residual nutrients from the culture medium, which does occur in submerged culture processes.
• In general, the industrial scaling of the surface culture processes over solid substrate results simpler than in submerged-culture processes.

## Claims

1. A process for biotransforming ferulic acid into vanillin, **characterized by** comprising the steps of:
(a) immobilizing a microorganism of the strain *Streptomyces setonii* ATCC 39116 in polyurethane foam;
(b) adding a solution of either ferulic acid, or any salt thereof;
(c) incubating for carrying out the biotransformation reaction;
(d) recovering the biotransformation solution obtained in step (c); and
(e) extracting the vanillin from the biotransformation solution obtained in step (d).

2. The biotransformation process in accordance with claim 1, **characterized in that** step (a) comprises the steps of:
(a) adding an inoculum of said strain *Streptomyces setonii* ATCC 39116 to a liquid culture medium;
(b) adding the inoculated culture medium to a polyurethane foam, for forming films between the interstices of said support;
(c) growing the strain *Streptomyces setonii* ATCC 39116 in the support; and
(d) eliminating the residual culture medium from said polyurethane foam.

3. The biotransformation process in accordance with claim 2, **characterized in that** the microorganism inoculum is grown under agitation at 190 min⁻¹, for a time period from 15 h to 30 h, at 37°C.

4. The biotransformation process in accordance with claim 2, **characterized in that** the inoculum volume that is added to the culture medium is from 3 % to 6 % of the total volume of the medium.

5. The biotransformation process in accordance with claim 2, **characterized in that** the liquid culture medium comprises:
glucose or maltose in a concentration ranging between 8 g L⁻¹ and 40 g L⁻¹; and
yeast extract in a concentration ranging between 1 g L⁻¹ and 15 g L⁻¹.

6. The biotransformation process in accordance with claim 2, **characterized in that** the volume of the inoculated culture medium that is added to the polyurethane foam is from 5 mL per gram of support to 50 mL per gram of support, preferably from 20 mL per gram of support to 35 mL per gram of support.

7. The biotransformation process in accordance with claim 2, **characterized in that** the height of the bed of the polyurethane foam is from 1 cm to 10 cm, preferably from 3 cm to 6 cm.

8. The biotransformation process in accordance with claim 2, **characterized in that** step (c) is carried out at a temperature of from 30 °C to 45 °C for a time period from 6 h to 35 h.

9. The biotransformation process in accordance with claim 2, **characterized in that** step (d) is carried out by compressing the polyurethane foam.

10. The biotransformation process in accordance with claim 1, **characterized in that** the solution of either ferulic acid or any salt thereof, of step (b), has a concentration from 5 g L⁻¹ to 30 g L⁻¹, preferably from 10 g L⁻¹ to 20 g L⁻¹.

11. The biotransformation process in accordance with claim 1, **characterized in that** the solution of either ferulic acid or any salt thereof, of step (b), has pH values between 7 and 9, preferably pH values between 7.5 and 8.5.

12. The biotransformation process in accordance with claim 1, **characterized in that** the incubation time for the biotransformation reaction of step (c) is from 7 h to 48 h, preferably from 15 h to 28 h.

13. The biotransformation process in accordance with claim 1, **characterized in that** the step (d) is carried out by compressing the polyurethane foam.

14. The biotransformation process in accordance with claim 1, **characterized in that** steps (b), (c) and (d) are carried out between 2 times to 15 times in a cyclic manner.

15. The biotransformation process in accordance with claim 1, **characterized in that** step (e) of extracting the vanillin from the biotransformation solution comprises the steps of:
(a) treating the biotransformation solution with activated coal or synthetic resins;
(b) eluding the vanillin adsorbed in the activated coal or in the synthetic resins with an alcoholic solution;
(c) concentrating by evaporation the eluded vanillin in the alcoholic solution; and
(d) crystallizing the vanillin in water.

16. The biotransformation process in accordance with claim 15, **characterized in that** the synthetic resins of step (a) are selected from the family of the amberlite.

17. The biotransformation process in accordance with claim 15, **characterized in that** the alcoholic solution of step (b) comprises a 95% solution of ethanol.

18. The biotransformation process in accordance with claim 15, **characterized in that** the step (c) of concentrating by evaporation the vanillin eluded in the alcoholic solution, is carried out at a temperature between 30 °C and 40 °C, and at reduced pressure.

## Patentansprüche

1. Verfahren zur Biotransformation von Ferulasäure in Vanillin, **gekennzeichnet durch** die folgenden Schritte:
(a) Immobilisieren eines Mikroorganismus des Stammes Streptomyces setonii ATCC 39116 in Polyurethanschaum;
(b) Hinzufügen einer Lösung entweder von Ferulasäure oder einem Salz davon;
(c) Inkubieren zur Durchführung der Biotransformationsreaktion;
(d) Rückgewinnen der in Schritt (c) erhaltenen Biotransformationslösung; und
(e) Extrahieren des Vanillins aus der Biotransformationslösung, erhalten in Schritt (d) .

2. Biotransformationsverfahren nach Anspruch 1, **dadurch gekennzeichnet, dass** Schritt (a) folgende Schritte umfasst:
(a) Hinzufügen eines Inokulums des Stammes Streptomyces setonii ATCC 39116 zu einem flüssigen Kulturmedium;
(b) Hinzufügen des inokulierten Kulturmediums zu einem Polyurethanschaum zur Bildung von Filmen zwischen den Zwischenräumen des Trägers;
(c) Züchten des Stammes Streptomyces setonii ATCC 39116 in dem Träger; und
(d) Entfernen des Restkulturmediums aus dem Polyurethanschaum.

3. Biotransformationsverfahren nach Anspruch 2, **dadurch gekennzeichnet, dass** das Mikroorganismus-Inokulum unter Rühren bei 190 min⁻¹, über einen Zeitraum von 15h bis 30h, bei 37°C gezüchtet wird.

4. Biotransformationsverfahren nach Anspruch 2, **dadurch gekennzeichnet, dass** das Inokulumvolumen, das dem Kulturmedium hinzugefügt wird, von 3% bis 6% des Gesamtvolumens des Mediums beträgt.

5. Biotransformationsverfahren nach Anspruch 2, **dadurch gekennzeichnet, dass** das flüssige Kulturmedium Folgendes umfasst:
- Glukose oder Maltose in einer Konzentration zwischen 8 g L⁻¹ und 40 g L⁻¹; und
- Hefeextrakt in einer Konzentration zwischen 1 g L⁻¹ and 15 g L⁻¹.

6. Biotransformationsverfahren nach Anspruch 2, **dadurch gekennzeichnet, dass** das Volumen des inokulierten Kulturmediums, das dem Polyurethanschaum hinzugefügt wird, von 5 mL pro Gramm des Trägers bis 50 mL pro Gramm des Trägers, vorzugsweise von 20 mL pro Gramm des Trägers bis 35 mL pro Gramm des Trägers beträgt.

7. Biotransformationsverfahren nach Anspruch 2, **dadurch gekennzeichnet, dass** die Höhe des Betts des Polyurethanschaums von 1 cm bis 10 cm, vorzugsweise 3 cm bis 6 cm beträgt.

8. Biotransformationsverfahren nach Anspruch 2, **dadurch gekennzeichnet, dass** Schritt (c) bei einer Temperatur von 30°C bis 45°C für einen Zeitraum von 6 h bis 35 h durchgeführt wird.

9. Biotransformationsverfahren nach Anspruch 2, **dadurch gekennzeichnet, dass** Schritt (d) durch Komprimieren des Polyurethanschaums durchgeführt wird.

10. Biotransformationsverfahren nach Anspruch 1, **dadurch gekennzeichnet, dass** die Lösung entweder von Ferulasäure oder einem Salz davon, in Schritt (b), eine Konzentration von 5 g L⁻¹ bis 30 g L⁻¹, vorzugweise von 10 g L⁻¹ bis 20 g L⁻¹ aufweist.

11. Biotransformationsverfahren nach Anspruch 1, **dadurch gekennzeichnet, dass** die Lösung entweder von Ferulasäure oder einem Salz davon, in Schritt (b), pH-Werte zwischen 7 und 9, vorzugsweise pH-Werte zwischen 7,5 und 8,5 aufweist.

12. Biotransformationsverfahren nach Anspruch 1, **dadurch gekennzeichnet, dass** die Inkubationszeit für die Biotransformationsreaktion in Schritt (c) von 7 h bis 48 h, vorzugsweise von 15 h bis 28 h beträgt.

13. Biotransformationsverfahren nach Anspruch 1, **dadurch gekennzeichnet, dass** Schritt (d) durch Komprimieren des Polyurethanschaums durchgeführt wird.

14. Biotransformationsverfahren nach Anspruch 1, **dadurch gekennzeichnet, dass** die Schritte (b), (c) und (d) zwischen 2-Mal bis 15-Mal in zyklischer Weise durchgeführt werden.

15. Biotransformationsverfahren nach Anspruch 1, **dadurch gekennzeichnet, dass** Schritt (e) des Extrahierens des Vanillins aus der Biotransformationslösung die folgenden Schritte umfasst:
(a) Behandeln der Biotransformationslösung mit Aktivkohle oder synthetischen Harzen;
(b) Ausweichen des in der Aktivkohle oder in den synthetischen Harzen adsorbierten Vanillins mit einer alkoholischen Lösung;
(c) Konzentrieren durch Verdampfen des entwichenen Vanillins in der alkoholischen Lösung; und
(d) Kristallisieren des Vanillins in Wasser.

16. Biotransformationsverfahren nach Anspruch 15, **dadurch gekennzeichnet, dass** die synthetischen Harze in Schritt (a) aus der Familie der Ambrelite ausgewählt werden.

17. Biotransformationsverfahren nach Anspruch 15, **dadurch gekennzeichnet, dass** die alkoholische Lösung in Schritt (b) eine 95%ige Lösung von Ethanol umfasst.

18. Biotransformationsverfahren nach Anspruch 15, **dadurch gekennzeichnet, dass** der Schritt (c) des Konzentrierens des in der alkoholischen Lösung entwichenen Vanillins durch Verdampfung bei einer Temperatur zwischen 30°C und 40°C und einem reduzierten Druck durchgeführt wird.

## Revendications

1. Procédé de biotransformation de l'acide férulique en vanilline, **caractérisé en ce qu'**il comprend les étapes :
(a) immobilisation d'un micro-organisme de la souche *Streptomyces setonii* ATCC 39116 dans de la mousse de polyuréthane ;
(b) addition d'une solution d'acide férulique ou d'un quelconque sel de celui-ci,
(c) incubation pour effectuer la réaction de biotransformation ;
(d) récupération de la solution de biotransformation obtenue à l'étape (c) ; et
(e) extraction de la vanilline de la solution de biotransformation obtenue à l'étape (d).

2. Procédé de biotransformation selon la revendication 1, **caractérisé en ce que** l'étape (a) comprend les étapes de :
(a) ajout d'un inoculum de ladite souche *Streptomyces setonii* ATCC 39116 à un milieu de culture liquide ;
(b) addition du milieu de culture inoculé à une mousse de polyuréthane pour former des films entre les interstices dudit support ;
(c) culture de la souche *Streptomyces setonii* ATCC 39116 dans le support ; et
(d) élimination du le milieu de culture résiduel de ladite mousse de polyuréthane.

3. Procédé de biotransformation selon la revendication 2, **caractérisé en ce que** l'inoculum de microorganismes est cultivé sous agitation à 190 min⁻¹, pendant une période de temps de 15 h à 30 h, à 37°C.

4. Procédé de biotransformation selon la revendication 2, **caractérisé en ce que** le volume d'inoculum qui est ajouté au milieu de culture est de 3% à 6% du volume total du milieu.

5. Procédé de biotransformation selon la revendication 2, **caractérisé en ce que** le milieu de culture liquide comprend du glucose ou du maltose dans une concentration comprise entre 8 g L⁻¹ et 40 g L⁻¹, et un extrait de levures dans une concentration comprise entre 1 g L⁻¹ et 15 g L⁻¹.

6. Procédé de biotransformation selon la revendication 2, **caractérisé en ce que** le volume du milieu de culture inoculé qui est ajouté à la mousse de polyuréthane est de 5 mL par gramme de support à 50 mL par gramme de support, de préférence de 20 mL par gramme de support à 35 mL par gramme de support.

7. Procédé de biotransformation selon la revendication 2, **caractérisé en ce que** la hauteur du lit de la mousse de polyuréthane est de 1 cm à 10 cm, de préférence de 3 cm à 6 cm.

8. Procédé de biotransformation selon la revendication 2, **caractérisé en ce que** l'étape (c) est réalisée à une température de 30 °C à 45°C pendant une période de temps de 6 h à 35 h.

9. Procédé de biotransformation selon la revendication 2, **caractérisé en ce que** l'étape (d) est réalisée par compression de la mousse de polyuréthane.

10. Procédé de biotransformation selon la revendication 1 **caractérisé en ce que** la solution d'acide férulique ou d'un quelconque sel de celui-ci, de l'étape (b), a une concentration de 5 g L⁻¹ à 30 g L⁻¹, de préférence de 10 g L⁻¹ à 20 g L⁻¹.

11. Procédé de biotransformation selon la revendication 1 **caractérisé en ce que** la solution d'acide férulique ou d'un quelconque sel de celui-ci, de l'étape (b) a des valeurs de pH entre 7 et 9, de préférence des valeurs de pH entre 7,5 et 8,5.

12. Procédé de biotransformation selon la revendication 1 **caractérisé en ce que** le temps d'incubation pour la réaction de biotransformation de l'étape (c) est de 7 h à 48 h, de préférence de 15 h à 28 h.

13. Procédé de biotransformation selon la revendication 1 **caractérisé en ce que** l'étape (d) est réalisée par compression de la mousse de polyuréthane.

14. Procédé de biotransformation selon la revendication 1, **caractérisé en ce que** les étapes (b), (c) et (d) sont réalisées entre 2 à 15 fois de manière cyclique.

15. Procédé de biotransformation selon la revendication 1, **caractérisé en ce que** l'étape (e) d'extraction de la vanilline de la solution de biotransformation comprend les étapes de :
(a) traitement de la solution de biotransformation avec du charbon activé ou des résines synthétiques;
(b) éluder la vanilline adsorbée dans le charbon activé ou dans les résines synthétiques avec une solution alcoolique;
(c) concentration par évaporation de la vanilline éludée dans la solution alcoolique; et
(d) cristallisation de la vanilline dans l'eau.

16. Procédé de biotransformation selon la revendication 15, **caractérisé en ce que** les résines synthétiques de l'étape (a) sont choisies dans la famille de l'amberlite.

17. Procédé de biotransformation selon la revendication 15, **caractérisé en ce que** la solution alcoolique de l'étape (b) comprend une solution à 95% d'éthanol.

18. Procédé de biotransformation selon la revendication 15, **caractérisé en ce que** l'étape (c) de concentration par évaporation de la vanilline éludée dans la solution alcoolique est réalisée à une température entre 30°C et 40°C, et à pression réduite.
